# FASCICULE DE BREVET EUROPEEN

(11) **EP 1 583 538 B1**
(45) Date de publication et mention de la délivrance du brevet: **30.07.2008**
(21) Numéro de dépôt: 04702722.2
(22) Date de dépôt: 16.01.2004
(51) Int. Cl.: A61K 31/57, A61P 25/00

(54) **UTILISATION DE LA 3-METHOXY-PREGNENOLONE POUR LA PRÉPARATION D'UN MÉDICAMENT DESTINÉ À TRAITER LES MALADIES NEURODEGENERATIVES**
VERWENDUNG VON 3-METHOXY-PREGNENOLON BEI DER HERSTELLUNG EINES ARZNEIMITTELS ZUR BEHANDLUNG VON NEURODEGENERATIVEN ERKRANKUNGEN
USE OF 3-METHOXY-PREGNENOLONE IN THE PRODUCTION OF A MEDICAMENT FOR TREATING NEURODEGENERATIVE DISEASES

(30) Priorité: 17.01.2003 FR 0300507
(43) Date de publication de la demande: 12.10.2005
(73) Titulaire: Mapreg, 94270 Le Kremlin Bicêtre (FR)
(72) Inventeur: BAULIEU, Etienne-Emile, F-92200 Neuilly-sur-Seine (FR); FELLOUS, Esther, F-75005 PARIS (FR); ROBEL, Paul, F-75015 PARIS (FR)
(74) Mandataire: Warcoin, Jacques
(86) Numéro de dépôt international: PCT/FR2004/000086
(87) Numéro de publication internationale: WO 2004/067010

(56) Documents cités:
- EP-A- 1 310 258
- WO-A-01/68068
- WO-A-02/36128
- US-B1- 6 245 757
- MURAKAMI K ET AL: "Pregnenolone binds to microtubule-associated protein 2 and stimulates microtubule assembly." PROCEEDINGS OF THE NATIONAL ACADEMY OF SCIENCES OF THE UNITED STATES OF AMERICA. UNITED STATES 28 MAR 2000, vol. 97, no. 7, 28 mars 2000 (2000-03-28), pages 3579-3584, XP000982542 ISSN: 0027-8424 cité dans la demande
- BURSI R.; GROEN M.B.: 'Application of (quantitative) structure-activity relationships to progestagens : from serendipity to structure-based design' J. MED. CHEM. vol. 35, 2000, pages 787 - 796

## Description

L'invention se rapporte à une nouvelle utilisation de dérivés de neurostéroïdes, notamment de la prégnènolone, pour le traitement des lésions aiguës ou chroniques du système nerveux, en particulier certaines maladies neurodégénératives, liée notamment à leur aptitude à stabiliser et /ou augmenter la polymérisation des microtubules neuronaux.

Une altération du cytosquelette des neurones est observée dans la plupart des lésions du SNC et des maladies neurodégénératives. Cette altération peut être la conséquence mais aussi la cause d'une souffrance des cellules affectées. En effet, une dépolymérisation des microtubules peut être directement responsable du dysfonctionnement de certains neurones et entraîner leur mort. De plus, ces altérations affectent le nombre et la longueur des prolongements neuritiques des cellules neuronales restantes et par conséquent diminue leur efficacité. Un traitement avec du NGF qui prévient l'atrophie dendritique permet une meilleure récupération fonctionnelle après une lésion du cortex cérébral chez le rat (Kolb et al., Neuroscience 1996). La dégradation du cytosquelette observée après un traumatisme du SNC (Zhang et al., J Neuropathol exp Neurol 2000) ou un épisode d'ischémie, résulte de nombreux facteurs, en particulier de l'augmentation du glutamate et du Ca⁺⁺ intracellulaire qui entraîne une dépolymérisation des microtubules, et de l'activation de protéases comme la Calpaïne qui dégradent MAP2. L'utilisation d'un inhibiteur de la calpaïne (Schumacher et al, J Neurochem 2000) et du relargage du glutamate (Springer et al., J Neurochem 1997) permet de diminuer les conséquences d'un traumatisme médullaire chez l'animal en préservant partiellement le cytosquelette.

La réparation des lésions récapitule le développement. L'existence de cellules souches dans certaines régions du système nerveux central est actuellement bien établie. Les lésions stimulent la prolifération de ces cellules. Cependant ces cellules doivent migrer et se différencier. La différenciation implique au premier chef le développement du cytosquelette.

Les protéines MAP2 représentent un des constituants majeurs des protéines associées aux microtubules neuronaux. Elles sont présentes dans toutes les extensions de ce qui constitue l'arborisation dendritique d'un neurone, arborisation dont on connaît l'importance pour l'établissement des connexions synaptiques (Matus, *Microtubules* 1994 ; Sanchez et al Prog.Neurobiol 2000). Les protéines MAP2 sont absolument requises pour la formation des dendrites comme le montrent les travaux d'auteurs qui, par la suppression de synthèse de MAP2 ont provoqué soit l'arrêt de croissance neuritique dans des neurones en culture (Caceres et al, Neuron 1992) soit l'arrêt de croissance dendritique chez des souris « knock out » pour le gène MAP2 (Harada et al, J.Cell.Biol. 2002). La synthèse des protéines MAP2 n'est pas à elle seule suffisante pour induire ce processus de croissance dendritique. Certains stéroïdes comme l'oestradiol ou la progestérone peuvent induire une augmentation de synthèse de MAP2 (Reyna-Neyra et al, Brain Res. 2002) sans induire de changements morphologiques spectaculaires. Par contre, certaines molécules se liant à MAP2 ont la propriété fort importante et originale de renforcer l'activité de cette protéine, à savoir son rôle dans l'activation du processus de polymérisation de la tubuline (Murakami et al, Proc Natl Acad Sci USA 2000) et l'établissement de structures microtubulaires d'une plus grande stabilité.

Il a été récemment montré que, après une ischémie cérébrale, des cellules souches pouvaient se différencier en neurones et s'intégrer dans les circuits neuronaux existant (Nakatomi et al., Cell 2002). La stimulation de la croissance des neurites de ces cellules par des molécules améliorant la polymérisation de la tubuline pourrait augmenter leur fonctionnalité.

Malgré de nombreuses recherches, aucune cible spécifique autre que des MAPs, n'a été identifiée à l'heure actuelle pour la PREG.

La protéine MAP2 est principalement présente dans les neurones. Les molécules se liant à MAP2 ont donc pour cible les cellules nerveuses. Ainsi, il est probable qu'elles n'ont pas d'action notable sur les autres types cellulaires dans lesquelles la concentration de MAP2 est très faible.

Les études montrant un effet de la prégnènolone (PREG) *in vivo* sont peu nombreuses mais sont en faveur d'un rôle bénéfique de ce stéroïde. Il a été montré que la PREG diminuait la réaction astrocytaire après une lésion cérébrale (Garcia-Estrada et al, Int J Devl Neuroscience 1999) et l'augmentation de la taille des astrocytes observée au cours du vieillissement (Legrand et Alonso, Brain Res 1998). Elle contribuait aussi à améliorer la récupération fonctionnelle après un traumatisme médullaire (Guth et al., Proc Natl Acad Sci USA 1994), La PREG protège des cellules issues d'une lignée hippocampique (HT-22) d'une toxicité induite par le glutamate et la protéine bêta amyloïde (Gursoy et al.; Neurochem Res 2001).

Il a de plus été démontré que la PREG ou un de ses dérivés substitué en position 3 par un groupement rapidement hydrolysable en radical hydroxyle sont utiles pour le traitement des maladies du système nerveux central et ce notamment en agissant sur les fonctions impliquant les microtubules (WO 01/68068 A, WO 02/36128 A et Murakami et al.; Proc Natl Acad Sci USA 2000, 97, 7, 3579-3584).

La PREG est le précurseur de toutes les hormones stéroïdes. Leur synthèse implique la conversion de la structure Δ5-3β-OH de la PREG en structure Δ4-3-céto (assurée par une enzyme dénommée 3βHSD). La Demanderesse a bloqué la structure Δ5-3β-OH pour empêcher son métabolisme et empêcher également la formation d'ester sulfate de PREG, molécule qui peut être neurotoxique à hautes concentrations. Ainsi, la Demanderesse a mis en évidence un composé, la 3-méthoxy-prégnènolone (3β-méthoxy-prégna-5-ène-20-one, en abrégé 3-méthoxy-PREG), qui possède cette propriété et qui de plus est au moins aussi actif que la PREG. La stabilité métabolique de ce composé a été vérifiée par spectrométrie de masse couplée à la chromatographie gazeuse.

La Demanderesse considère ainsi que l'invention se rapporte à la 3-méthoxy-PREG, mais aussi à toutes les molécules dérivées de la prégnènolone, portant une fonction 3-méthoxy ou présentant une fonction 3' pouvant être convertie en 3-méthyl-éther). Ces molécules sont alors incapables de se convertir en métabolites pourvus d'activités progestatives (la progestérone est un métabolite directe de la PREG et, en plus de son activité hormonale, elle est un antagoniste de la PREG pour la polymérisation des microtubules), androgéniques, estrogéniques et glucocorticoïdes. Elles ne peuvent pas également se convertir en esters sulfates qui, à l'instar du sulfate de PREG, pourraient avoir des effets neurotoxiques.

Dans le cadre de la présente invention, la Demanderesse a mis en évidence le fait que la 3-méthoxy-PREG, ou d'autres molécules selon l'invention peuvent jouer un rôle majeur dans la polymérisation et/ou la stabilisation des microtubules, et présente des activités tout à fait remarquables, pour le traitement de pathologies liées au système nerveux.

Par "pathologies liées au système nerveux", on entend les pathologies liées au système nerveux central ou périphérique, particulièrement celles dans lesquelles les microtubules neurocellulaires sont affectés.

La 3-méthoxy-PREG présente la formule suivante :

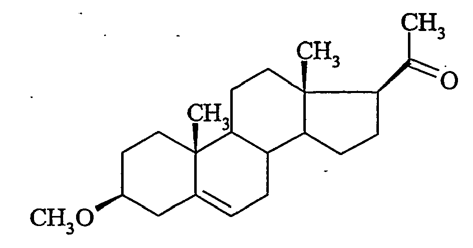

Ainsi, l'invention se rapporte à l'utilisation de la 3-méthoxy-PREG ou d'une molécule dérivée de la prégnènolone portant une fonction 3-méthoxy et incapable de se convertir en métabolite ou esters sulfate de prégnènolone, pour la préparation d'un médicament destiné à stimuler la polymérisation et/ou la stabilisation des microtubules pour traiter une lésion aiguë ou chronique ou une maladie dégénérative du système nerveux, ladite molécule présentant la formule générale I : 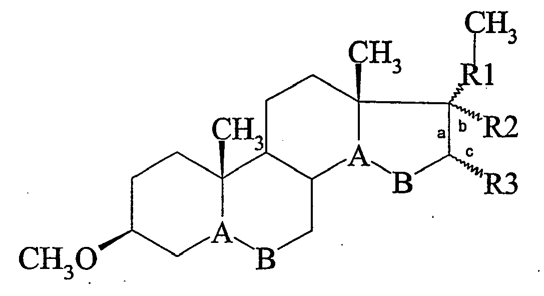 dans laquelle : 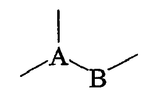 représente 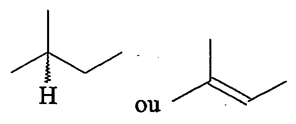
R1 = -CO- ; -CH(OH)- ou -CH(O-COCH₃)-
R2 = H ou CHCl₂,
R3 = H ou CH₃, ou
R2 et R3 forment ensemble un cycle :

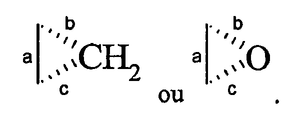

Dans un mode de réalisation préféré, ladite molécule est la 3-méthoxy-PREG (3β-méthoxy-prégna-5-ène-20-one).

Dans un autre mode de réalisation, ladite molécule est la 3β-méthoxy-prégna-5-ène-20-one-17-α-dichlorométhyle.

Dans un autre mode de réalisation, ladite molécule est la 3β-méthoxy-5α-prégnane-20-one.

Dans un autre mode de réalisation, ladite molécule est la 3β-méthoxy-5α-prégnane-20β-ol.

Dans un autre mode de réalisation, ladite molécule est la 3β-méthoxy-PREG-16α-méthyle.

Dans un autre mode de réalisation, ladite molécule est la 3β-méthoxy-PREG-16β-méthyle.

Dans un autre mode de réalisation, ladite molécule est la 3β-méthoxy-prégna-5,14-diène-20-one.

Dans un autre mode de réalisation, ladite molécule est la 3β-méthoxy-PREG-16α,17α-époxy.

Dans un autre mode de réalisation, ladite molécule est la 3β-méthoxy-PREG-16α, 17α-méthylène.

Dans un autre mode de réalisation, ladite molécule est la 3β-méthoxy-Prégna-5-ène-3β,20β-diol-20-acétate.

Dans un autre mode de réalisation, ladite molécule est la 3β-méthoxy-5α-prégnane-20-one-16α-méthyle.

La 3-méthoxy-PREG peut, dans le cadre de la présente invention être utilisée pour préparer un médicament utile pour le traitement d'autres syndromes comme le ralentissement mental et la perte de concentration, la douleur incluant, la douleur aiguë, la douleur post-opératoire, la douleur chronique, la douleur nociceptive, la douleur neuropathique, les syndromes de douleur psychogéniques, la douleur associée aux neuropathies périphériques, certains états psychiatriques (notamment les états dépressifs), des épisodes dissociatifs incluant l'amnésie dissociative, la fugue dissociative et le désordre d'identité dissociatif, d'autres maladies neurodégénératives comme la maladie de Parkinson, la maladie d'Huntington, les maladies liées aux prions, la Sclérose Latérale Amyotrophique (SLA), la sclérose en plaques.

D'une façon générale, on utilise la 3-méthoxy-PREG ou la molécule dérivée selon l'invention pour le traitement de toute maladie pour laquelle une polymérisation et/ou une stabilisation accrue des microtubules est recherchée ou bénéfique.

Dans un mode de réalisation préféré selon l'invention, ladite maladie est choisie dans le groupe constitué de la maladie d'Alzheimer, la maladie de Parkinson, la perte de mémoire induite par l'âge, la perte de mémoire induite par la prise de substances, une lésion traumatique, une lésion cérébrale, une lésion de la moelle épinière, en particulier une compression médullaire, l'ischémie, la douleur, notamment la douleur névritique, la dégénérescence nerveuse, et la sclérose en plaques.

Dans un mode de réalisation préféré, et notamment pour le traitement des maladies liées à un dérèglement du système nerveux central, ledit médicament comprend également un excipient ou un composé permettant de formuler ladite 3-méthoxy-PREG pour permettre une meilleure traversée de la barrière hémato-encéphalique. Un tel excipient ou composé peut aussi permettre une traversée plus rapide ou plus durable de ladite barrière hémato-encéphalique.

Un tel excipient ou composé peut être un peptide, tels les peptides décrits dans la demande WO 00/32236, ou de la 2-pyrrolidone.

Les compositions pharmaceutiques utilisées dans l'invention peuvent être administrées par n'importe quelle voie d'administration incluant, mais sans se limiter, la voie orale, intraveineuse, intramusculaire, intra artérielle, intra médullaire intra ventriculaire, transdermique, sous-cutanée, intra péritonéale, intra nasale, entérale, topique, sublinguale, ou rectale.

On peut envisager un traitement continu ou à long terme directement effectué par l'intermédiaire du liquide cérébro-spinal en utilisant une pompe implantée dans l'espace sub-arachnoïdien du cerveau ou de la moelle épinière. Un tel implant pourrait contenir une solution concentrée de 3-méthoxy-PREG (par exemple de l'isopropyl-beta-cyclodextrine diluée avec du liquide céphalo-rachidien artificiel)

De plus, la 3-méthoxy-PREG peut être administrée avec d'autres composés d'agents biologiquement actifs (par exemple des tensioactifs, des excipients, des transporteurs, des diluants et/ou des véhicules pharmaceutiquement acceptables). Ces composés sont bien connus de l'homme du métier. On peut trouver des détails sur ces produits dans la dernière édition de Remington's Pharmaceutical Sciences (Maack Publishing Co., Easton, Pa.).

Dans les compositions pharmaceutiques de la présente invention pour l'administration orale, sublinguale, sous-cutanée, intramusculaire, intraveineuse, transdermique, locale ou rectale, l'ingrédient actif (3-méthoxy-PREG ou molécule dérivée) peut être administré sous formes unitaires d'administration, en mélange avec des supports pharmaceutiques classiques, applicables aux animaux ou aux êtres humains. Les formes unitaires d'administration appropriés comprennent les formes par voie orale telles que les comprimés, les comprimés enrobés, les dragées, les gélules et capsules de gélatine molle, les poudres, les granules et les solutions ou suspensions orales, les formes d'administration sublinguale et buccale, les formes d'administration sous-cutanée, intramusculaire, intraveineuse, intra nasale ou intraoculaire et les formes d'administration rectale.

Les compositions pharmaceutiques peuvent également contenir des conservateurs, solubilisants, stabilisants, agents mouillants, émulsifiants, édulcorants, colorants, arômes, des sels destinés à modifier la pression osmotique, des tampons, des correcteurs de goût ou des antioxydants. Elles peuvent également contenir d'autres substances thérapeutiquement activés.

Ainsi, les compositions pharmaceutiques selon l'invention peuvent également contenir d'autres agents neuroprotecteurs, notamment, des composés qui augmentent-la régénération neuronale. De tels agents peuvent être en particulier choisis parmi les facteurs de croissance des neurones comme les facteurs de croissance des fibroblastes (FGF), acide ou basique, FGF-3, FGF-4, FGF-6, ou le facteur de croissance des kératinocytes (KGF). On peut également envisager l'ajout d'un agent neuroprotecteur tel le facteur de croissance nerveux (NGF), le facteur neurotrophique dérivé du cerveau (BDNF), la neurotrophine 3 ou 4, le TGF-beta.1, les interleukines, les facteurs de croissance dérivés de l'insuline (IGFs).

On peut aussi utiliser tout autre type d'agents thérapeutiques antioxydants et neuroprotecteurs, notamment les inhibiteurs du glutamate.

Lorsque l'on prépare une composition solide sous forme de comprimés, on mélange l'ingrédient actif principal avec un véhicule pharmaceutique tel que la gélatine, l'amidon, le lactose, l'acide stéarique ou le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures, avec comme excipient des huiles végétales, des cires, des graisses, des polyols semi-solides ou liquides etc...

Une préparation sous forme de sirop ou d'élixir peut contenir l'ingrédient actif conjointement avec un édulcorant, un antiseptique, ainsi qu'un agent donnant du goût et un colorant approprié. On utilise des excipients tels l'eau, les polyols, le saccharose, le sucre inverti, le glucose...

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyols semi-solides ou liquides tels les poly-éthylène-glycols, des cires, des huiles naturelles ou hydrogénées, des graisses...

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles. On peut utiliser, comme excipient, l'eau, des alcools, des polyols, le glycérol, des huiles végétales...

Le principe actif peut être formulé également sous forme de microcapsules, éventuellement avec un ou plusieurs supports additifs.

Pour le traitement de la douleur, la voie d'application topique est la voie d'administration préférée. Ici, les compositions selon l'invention peuvent être présentées sous forme d'un gel, d'une pâte, d'un onguent, d'une crème, d'une lotion, d'une suspension liquide aqueuse, aqueuse-alcool, d'une solution huileuse, d'une dispersion du type lotion ou sérum, d'un gel anhydre ou lipophile, ou d'une émulsion de consistance liquide ou semi-solide de type lait, obtenue en dispersant une phase grasse dans une phase aqueuse ou vice versa, ou de suspensions ou les émulsions de cohérence(consistance) douce, semi-solide de la crème ou le type de gel, ou alternativement de microémulsions, de microcapsules, de microparticules ou de dispersions vésiculaires au type ionique et-ou nonionique. Ces compositions sont préparées selon des méthodes standard.

De plus, un tensioactif peut être inclus dans la composition afin de permettre une pénétration plus profonde de la 3-méthoxy-PREG.

Parmi les ingrédients envisagés, l'invention comprend les agents permettant une augmentation de pénétration choisis par exemple dans le groupe consistant en l'huile minérale, l'éthanol, la triacétine, la glycérine et le glycol de propylène; des agents de cohésion sont choisis par exemple dans le groupe consistant en le polyisobutylène, l'acétate de polyvinyle, l'alcool de polyvinyle et les agents d'épaississement.

Ainsi, dans un mode de réalisation préféré selon l'invention, ledit médicament se présente sous forme injectable.

Dans un autre mode de réalisation préféré selon l'invention, ledit médicament se présente sous forme permettant une prise orale.

D'une manière préférée, ledit médicament comprend une quantité efficace de 3-méthoxy-PREG, en particulier comprise entre 50 et 2500 mg par voie parentérale.

Ledit médicament comprend préférentiellement une quantité efficace de 3-méthoxy-PREG ou de molécule dérivée de la prégnènolone présentant une fonction 3-méthoxy, telle que la quantité administrée au patient soit comprise entre 1 et 100 mg / kg.

Une quantité efficace de 3-méthoxy-PREG est une quantité qui permet, au sens de la présente invention, la stabilisation et/ou polymérisation des microtubules après administration à l'hôte. Ainsi, l'administration d'une quantité efficace de 3-méthoxy-PREG aboutit à l'amélioration ou l'élimination de la maladie. La quantité de 3-méthoxy-PREG administrée à l'hôte variera en fonction de facteurs, y compris la taille, l'âge, le poids, la santé générale, le sexe, le régime alimentaire et le moment de l'administration, la durée ou les particularités de la maladie associée à la dépolymérisation/déstabilisation des microtubules. Par exemple, le dosage devra réaliser, au niveau du site d'action, une concentration de l'ordre de 0,5 à 100 µM. L'ajustement des doses est bien connu de l'homme du métier.

L'invention se rapporte donc à une utilisation thérapeutique de la 3-méthoxy-PREG. L'invention se rapporte donc à ce composé en tant que médicament.

Une composition pharmaceutique, comprenant de la 3-méthoxy-PREG, ou un composé dérivé de la prégnènolone ayant une fonction 3-méthoxy de formule générale I, en tant que principe actif, et un excipient pharmaceutiquement acceptable, est également un objet de l'invention.

La Demanderesse a mis en évidence l'activité de la 3-méthoxy-PREG pour stabiliser et/ou induire la polymérisation des microtubules d'une cellule.

Ainsi, d'une façon plus générale, l'invention se rapporte à une méthode pour augmenter la stabilisation et/ou induire la polymérisation des microtubules dans une cellule comprenant l'étape d'exposer ladite cellule en présence de 3-méthoxy-PREG, à une concentration d'environ 0,5 à 100 µM, de préférence 0,5 à 50 µM. La polymérisation des microtubules peut être indiquée et repérée par un immuno-marquage de la protéine MAP2 associée à ces microtubules. De façon préférée, cette méthode est mise en oeuvre *in vitro* ou *ex vivo* (sur des cellules isolées d'un patient, traitées *in vitro* et réinjectées) dans certains cas.

L'invention se rapporte également à une méthode pour augmenter la pousse des neurites dans une cellule comprenant l'étape d'exposer ladite cellule en présence de 3-méthoxy-PREG, à une concentration d'environ 0,5 à 50 µM. Cette méthode est également mise en oeuvre *in vitro* de façon préférée.

L'invention a également pour objet une méthode pour réduire la dépolymérisation des microtubules et/ou la rétractation des neurites dans une cellule, comprenant l'étape d'exposer ladite cellule en présence de 3-méthoxy-PREG, à une concentration d'environ 0,5 à 50 µM. Cette méthode est également mise en oeuvre *in vitro* de façon préférée.

### DESCRIPTION DES FIGURES

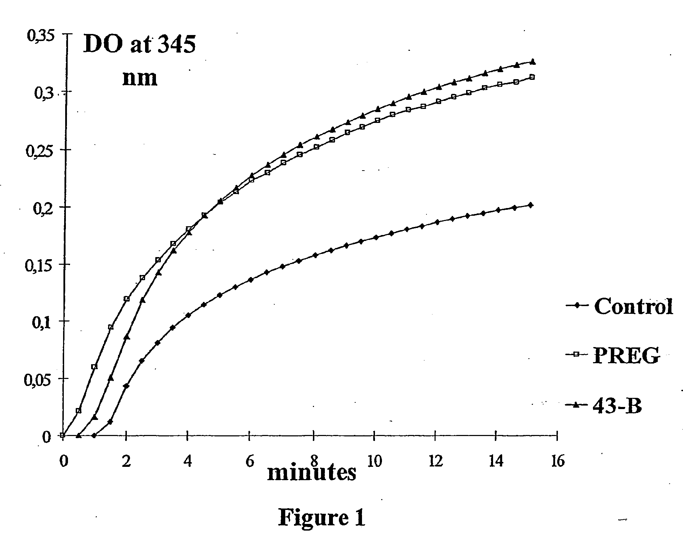

Figure 1: Cinétique de polymérisation des microtubules *in vitro*: effets de la PREG (prégnènolone) et de la molécule 43B (3-méthoxy-PREG). Un mélange de MAP2 et de tubuline purifiées est effectué en présence de GTP à 4°C dans une cuvette de spectrophotomètre. La polymérisation est induite par chauffage à 37°C et suivie par l'augmentation de densité optique (DO) indicatrice de la quantité de polymères formés. En présence de PREG et de la molécule 43B, le temps de latence est diminué alors que la vitesse de polymérisation et la quantité de microtubules sont nettement augmentées comparativement à la cinétique témoin (control) en présence de solvant seul.

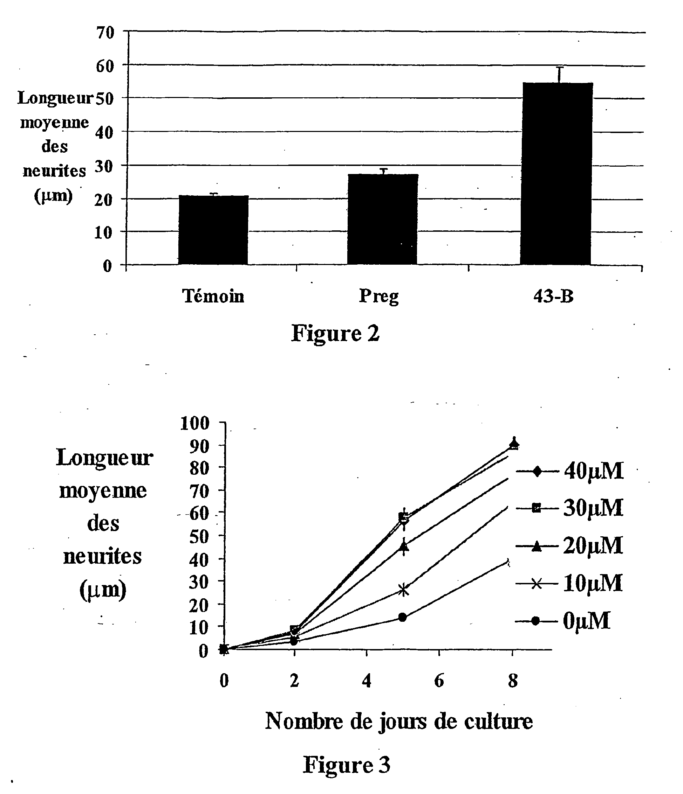

Figure 2 : Effet de la PREG et de la 3-méthoxy-PREG (43B) sur la longueur moyenne des neurites des cellules PC12. Les cellules PC12 ont été cultivées pendant 3 jours en présence de NGF (10 ng/ml) avec ou sans (témoin) addition des molécules PREG ou 43B (30µM). Chaque molécule a été testée sur trois puits de culture. Les mesures ont été effectuées à l'aide du logiciel Scion Image sur 200 cellules par puit.
Figure 3 : Relation dose-effet de la molécule 43B sur la longueur moyenne des neurites des cellules PC12. Les cellules PC12 ont été cultivées en présence de NGF (10 ng/ml) et de concentrations croissantes de 3-méthoxy-PREG (43B). La longueur des neurites a été mesurée sur 200 cellules par puit après 2, 5 et 8 jours de culture.

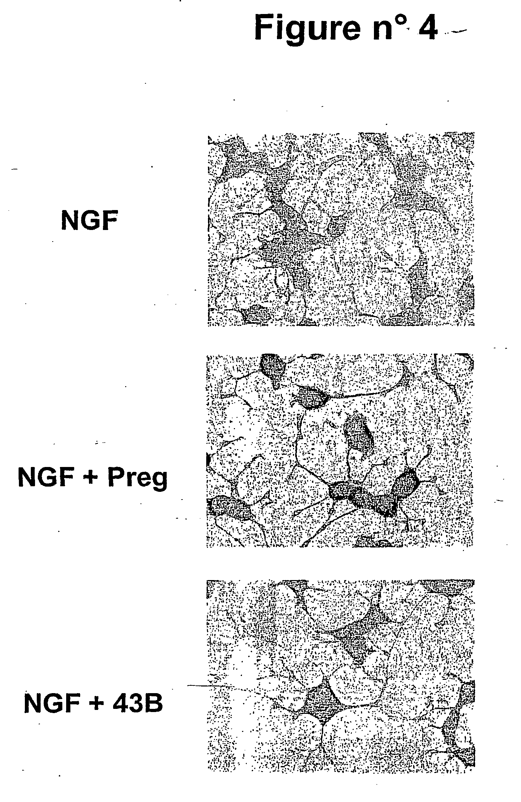

Figure 4 : Immuno-marquage de MAP2 associé aux microtubules des cellules PC-12 traitées avec PREG ou 3-méthoxy-PREG. Les cellules PC12 ont été cultivées en présence de NGF (10 ng/ml) et de PREG ou 3-méthoxy-PREG (20 µM). Elles ont été fixées et exposées à des anticorps anti-MAP2 qui visualisent exclusivement MAP2 associée aux microtubules.

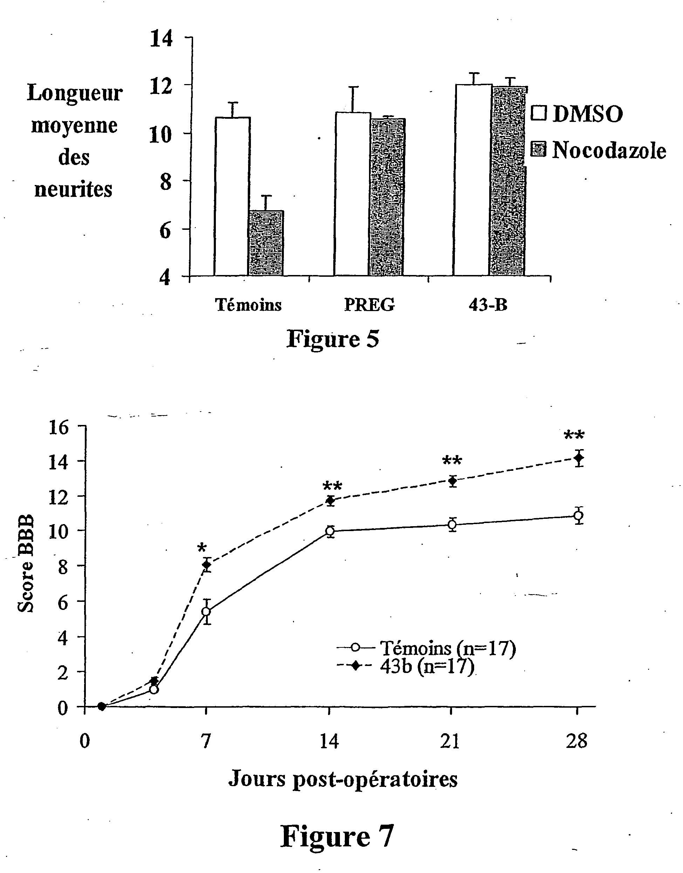

Figure 5 : Rétraction des neurites induite par le nocodazole. Après 7 jours de culture en présence de NGF (10 ng/ml), les cellules ont été prétraitées pendant une heure avec la PREG (30µM) ou 43B (30µM), puis exposées au nocodazole pendant 15 min. (colonnes blanches : solvant DMSO seul ; colonnes grises: Nocodazole).

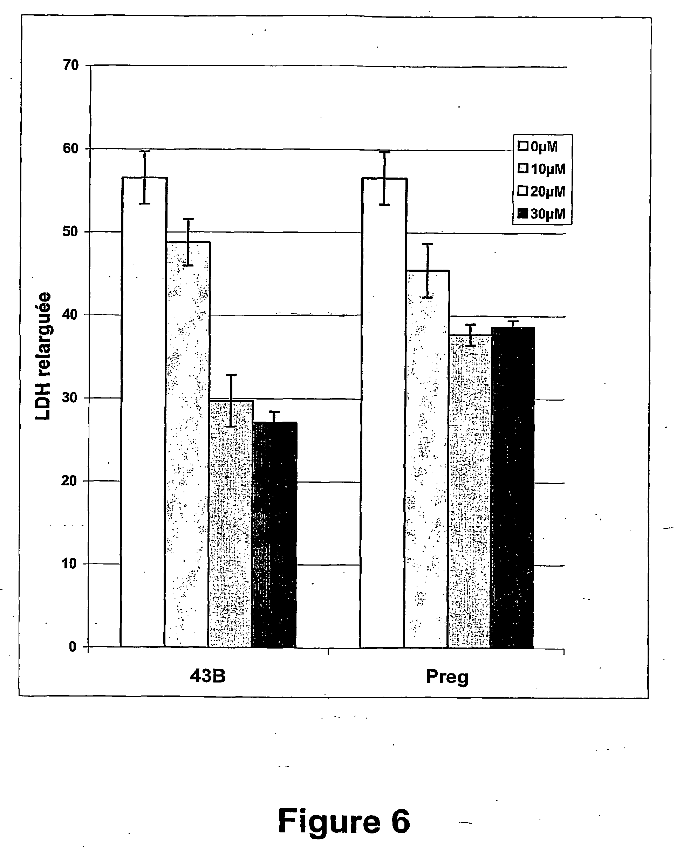

Figure 6: Toxicité de l'acide okadaïque. Protection par les stéroïdes. Les cellules de neuroblastome humain SH SY5Y ont été cultivées pendant 24 heures en présence d' acide okadaïque seul ou associé à des concentrations croissantes de PREG ou de 3-méthoxy-PREG. La mortalité cellulaire a été indiquée par la libération de lactate déshydrogénase (LDH) dans le milieu de culture.
Figure 7: Effet de la molécule 43B sur la récupération locomotrice des rats après une compression médullaire. La locomotion des animaux a été évaluée en aveugle dans l'intervalle 1-28 jours après l'opération par le score BBB qui évalue le degré des paralysies (il est d'autant plus élevé que la récupération est meilleure). Signification statistique : * : p<0.001 ; ** : p<0.0001

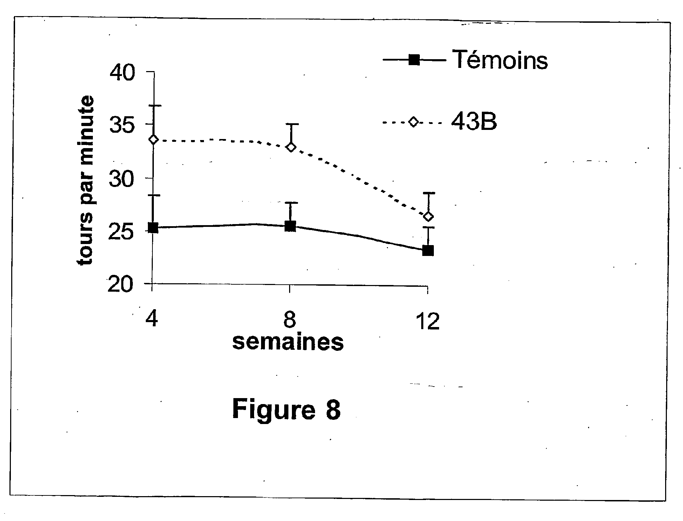

Figure 8: Effet de la molécule 43B sur l'incoordination motrice de souris transgéniques exprimant l'isoforme la plus longue de la protéine tau humaine dans leurs neurones.
   L'incoordination motrice a été évaluée en aveugle pendant 12 semaines après insertion sous-cutanée d'implant vide ou contenant soit de la PREG soit de la 3-méthoxy-PREG (43B). La vitesse maximum de rotation du tambour de Rotarod avant la chute est une mesure de la coordination motrice (elle est d'autant plus grande que l'effet du traitement est meilleur).

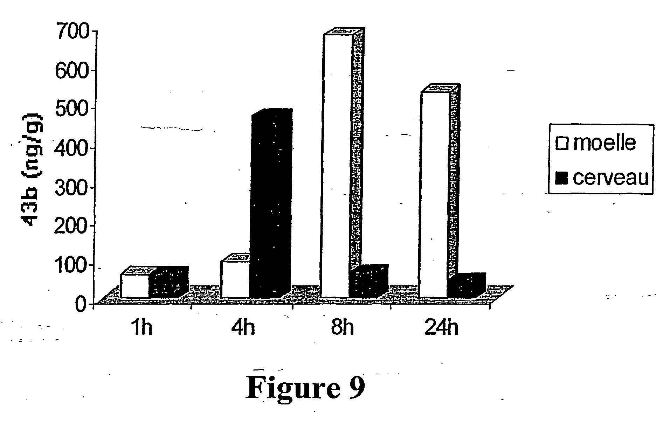

Figure 9 : Cinétique d'apparition de la 3-méthoxy-PREG (43B) dans le cerveau et la moelle épinière des rats après une injection sous cutanée (12mg/kg) de 43B en solution dans de l'huile de sésame.

Les exemples qui suivent sont destinés à illustrer l'invention.

### EXEMPLES

### Exemple 1 : synthèse de la 3-méthoxy-PREG

10 g (52 mmol) de p-toluènesulfonyl chloride sont ajoutés à une solution de 5g (15,8 mmol) de prégnènolone dans 30 ml de pyridine. L'ensemble est laissé sous agitation pendant 14 heures, puis versé dans 100 ml d'eau distillée. Après refroidissement du milieu réactionnel à 0°C, l'ensemble est filtré et le solide blanc obtenu est séché sous vide pour fournir 7,4 g (98%) de tosylate-de prégnènolone.

Les 7,4 g de tosylate de prégnènolone sont portés au reflux du méthanol (50 ml) pendant 4 heures. Après refroidissement et évaporation du solvant, le brut réactionnel est repris dans 100 ml d'éthyle et lavé 3 fois avec 100 ml d'une solution 10 % de bicarbonate de sodium. Après séchage de la phase organique sur Na₂SO₄, celle-ci est évaporée à sec sous pression réduite pour fournir 5,2g (100%) de 3-méthoxy-PREG sous forme d'une poudre blanche.

Une nouvelle synthèse de 3-méthoxy-PREG a été réalisée à l'échelle du kilogramme. La pureté du produit final a été confirmée par RMN et était supérieure à 97,5 %, avec un seul contaminant mineur facilement séparable par HPLC.

La prégnènolone peut être obtenue à bas prix de sources commerciales.

### Exemple 2: Test d'activité de la 3-méthoxy-PREG. Comparaison avec la PREG

Ce test mesure *in vitro* l'effet de molécules sur la polymérisation des microtubules induite par MAP2. Cette polymérisation se produit quand des proteines MAP2 et de la tubuline sont mélangées à des concentrations adéquates en présence de GTP. Elle s'accompagne d'une augmentation de la densité optique mesurée à 345 nm pendant 15 à 30 minutes avec un spectrophotomètre UNICON thermostaté à 37°C (figure 1).

On voit que la molécule 43B, correspondant à la 3-méthoxy-PREG, active la polymérisation des microtubules comme la prégnènolone (PREG). D'autres molécules, comme la progestérone et le sulfate de prégnènolone, ne la stimulent pas mais sont antagonistes de la PREG (non montré).

### Exemple 3 : modèles cellulaires

### Effet des molécules sur la croissance neuritique

Pour tester l'effet sur la croissance neuritique de molécules selectionnées, nous avons utilisé dans un premier temps la lignée PC12 qui est employée depuis longtemps pour de nombreuses recherches en neurobiologie. Les cellules de cette lignée, issue d'un phéochromocytome de rat, forment, en présence de NGF (Nerve Growth Factor, facteur de croissance nerveux), des prolongements neuritiques contenant des microtubules auxquels sont associées des MAPs. La croissance de ces prolongements est stimulée par l'addition de PREG. En présence de PREG [30µM], l'augmentation de la longueur moyenne des neurites après 4 jours de culture atteint 60%. Le screening d'autres stéroïdes naturels ou synthétiques de synthèse a permis de sélectionner plusieurs molécules présentant des effets supérieurs à ceux de la PREG (Figure 2). En particulier, l'addition de la molécule 43B, qui peut être facilement synthétisée à partir de la PREG, entraîne une augmentation spectaculaire (pouvant atteindre 500%) de la longueur des neurites formés en présence de NGF (Figure 3). Cette croissance de neurites accompagne la stimulation par 43-B de l'association de MAP2 aux microtubules Figure 4).

### Effet des stéroïdes sur la résistance des microtubules au nocodazole

Le nocodazole est un agent dépolymérisant des microtubules. Son addition à des cultures de cellules PC12, différenciées en présence de NGF, entraîne une rétraction des neurites résultant de la dépolymérisation de leurs microtubules. Le prétraitement des cellules par la PREG ou par 43B rend les neurites résistants au nocodazole, en raison d'une augmentation de la stabilité des microtubules, condition nécessaire à la formation de longs neurites. (Figure 5).

### Effet des stéroïdes sur la cyto-toxicité de l'acide okadaïque

L'acide okadaïque est un inhibiteur des phosphatases de protéines. Or l'hyperphosphorylation de la protéine tau est impliquée dans la dépolymérisation des microtubules et dans la mort des cellules du neuroblastome humain SH SY5Y. L'exposition de cultures de cellules SY5Y à l'acide okadaïque entraîne effectivement une importante mortalité cellulaire. Celle-ci est diminuée par l'addition simultanée de PREG et bien plus fortement par celle de 3-méthoxy-PREG (43 B) (figure 6).

### Exemple 4 : Tests de toxicité

### Toxicité cellulaire

Les tests de toxicité cellulaire sont réalisés en routine sur la lignée cellulaire PC12. Les premiers résultats obtenus montrent que la PREG et le 43B ne présentent pas de toxicité à des concentrations allant jusqu'à leur limite de solubilité (environ 50µM).

### Toxicité in vivo

L'injection journalière pendant 1 mois de 48mg/kg de 43B (soit 4 fois la dose active dans les traumatismes médullaires) n'a affecté ni le poids moyen ni le comportement des rats.

### Exemple 5 : Expériences in vivo - Traumatismes médullaires

### Modèle de contusion médullaire

Pour déterminer les effets neuroprotecteurs des molécules testées, un modèle de compression médullaire est utilisé. Ce modèle entraîne une paralysie totale des animaux les premiers jours suivant l'opération. Cette période de paralysie est suivie d'une phase d'environ trois semaines pendant laquelle les animaux récupèrent partiellement leur fonction motrice. L'étude de cette récupération à l'aide d'un test fonctionnel simple et précis reposant sur l'observation des animaux (BBB score) permet d'étudier la vitesse et le degré de récupération des animaux avec ou sans traitement.

Deux groupes de rats ont subi une compression médullaire. Les animaux ont ensuite reçu pendant 2 semaines, une injection sous-cutanée quotidienne contenant, soit de l'huile de sésame seule (groupe témoin, n=20), soit de l'huile de sésame contenant la molécule 43B (groupe 43B, n=20 ; 12mg/kg/jour). La première injection a été effectuée 5 min après la compression médullaire. La locomotion des animaux a été évaluée en aveugle 1, 4, 7, 14, 21 et 28 jours après l'opération par le score BBB. Trois animaux ont dû être exclus de l'étude dans chaque groupe. L'analyse statistique des résultats par le test non paramétrique de Mann-Whitney montre que les animaux traités avec 43B présentent des résultats très significativement supérieurs aux animaux témoins dès le septième jour après l'opération (Figure 7).

### Exemple 6 : Expériences in vivo - Ischémie cérébrale

Deux modèles d'ischémie cérébrale chez le rat ont été développés.

Le premier est un modèle d'ischémie focale permanente ou transitoire de l'artère cérébrale moyenne par électrocoagulation ou clampage (évaluation de la neuroprotection par quantification du volume de la zone lésée).

Le deuxième est un modèle d'ischémie cérébrale globale et transitoire. Ce modèle est réalisé chez le rat en électrocoagulant et sectionnant les artères vertébrales puis en clampant les artères carotides pendant une durée de 15 min (évaluation de la neuroprotection et de l'augmentation de la plasticité cérébrale par quantification de la perte neuronale dans la zone CA1 de l'hippocampe et par des tests de mémoire).

### Exemple 7 : Expériences in vivo - Modèle de maladie neurodégénérative de type Alzheimer (souris transgéniques)

Afin d'évaluer le potentiel thérapeutique de 43B pour le traitement des maladies neurodégénératives de type Alzheimer, on peut utiliser une lignée homozygote de souris transgéniques, telles les souris décrites par Götz, (EMBO J. 1995;14(7):1304-13).

Ces souris expriment l'isoforme humaine la plus longue de la protéine tau. Elles présentent des symptômes de dysfonctionnement neurologique qui se traduisent par une faiblesse musculaire et une diminution de la coordination motrice corrélées histologiquement à l'apparition de neurites anormaux et de protéines tau hyperphosphorylées comme dans la maladie d'Alzheimer. Cette phosphorylation pathologique diminue l'affinité de tau pour les microtubules et favorise son agrégation.

En traitant ces souris avec des molécules qui augmentent la stabilité des microtubules, on cherche à augmenter la proportion de protéine tau associée aux microtubules et de retarder ainsi l'apparition des symptômes.

Un élevage de ces souris a été entrepris à Bicêtre et un génotypage a permis de sélectionner les souris homozygotes pour le transgène. A l'âge de 21± 3 jours, 3 groupes de 10 souris ont été constitués, recevant respectivement en sous-cutané un implant vide, un implant de PREG ou un implant de 3-méthoxy-PREG. Ces implants libèrent 0,38 mg de stéroïde par jour pendant 90 jours. La progression de l'incoordination motrice a été suivie par des tests répétés sur Rotarod. Seule la 3-méthoxy-PREG a eu un effet bénéfique sur les troubles moteurs (Figure 8).

### Exemple 8 : Expériences in vivo - Performance mnésique

### Déficit mnésique induit par la colchicine

La colchicine, une substance qui dépolymérise les microtubules sans bloquer la synthèse protéique, est injectée à des doses faibles qui n'induisent pas de mort neuronal dans l'hippocampe de rat. Ces injections entraînent un déficit d'apprentissage résultant d'une dépolymérisation durable des microtubules. L'objectif est de tester l'effet des molécules stabilisatrices des microtubules sur les déficits mnésiques et les lésions histologiques de l'hippocampe induits par la colchicine.

### Déficit mnésiques au cours du vieillissement

Les études sur le vieillissement sont réalisées sur des rats âgés présentant des déficits mnésiques. L'objectif de cette expérimentation est de pallier ces déficits par un traitement chronique avec nos molécules.

Les expériences de mémoire en deux étapes sont basées sur l'exploration spontanée de la nouveauté et adaptées des expériences décrites par Dellu et al. (1992, Brain Res., 588, 132-9) et Ladurelle et al. (2000, Brain Res., 858, 371-9). Les enseignements techniques de ces deux publications concernant les tests de mémoire spatiale utilisant des labyrinthes sont inclus par référence à la présente demande.

### Exemple 9 : Pharmacocinétique

La pharmacocinétique des molécules testées *in vivo* est réalisée par des dosages effectués par chromatographie en phase gazeuse couplée à la spectrométrie de masse (GCMS)

Une étude a été réalisée avec la PREG et la molécule 43B. Son principal objectif était de démontrer que la molécule 43B passait la barrière hémato-encéphalique.

On a injecté les rats soit avec la PREG, soit avec 43B diluées dans de l'huile de sésame et dosé par GCMS la quantité de PREG ou de 43B dans différents organes 1h, 4h ,8h et 24h après l'injection (12mg/kg dans 0.5ml d'huile de sésame ; injection en sous-cutanée).

Les résultats présentés figure 9 montrent que la molécule 43B pénètre rapidement dans la moelle épinière et le cerveau des rats injectés, et tend à s'y accumuler.

Ces résultats obtenus *in vitro* et *in vivo* montrent bien que la molécule 43B (3-méthoxy-PREG) donne des résultats spectaculaires sur la croissance des neurites en culture et sur le modèle de compression médullaire.

### Exemple 10 : autres molécules selon l'invention

Les indices de liaison et d'activité sont exprimés en % par rapport à la PREG.

La liaison (affinité) est mesurée par déplacement de la PREG-³H.

L'activité est mesurée par l'augmentation de densité optique à 345nm d'un mélange de tubuline et de MAP2 purifiées, incubées à 37°C en présence de GTP.

La stimulation de la pousse neuritique est effectuée sur les cellules PC12 différenciées en présence de NGF 10ng/ml et du stéroïde à tester 30 µM pendant 3 jours. Pour chaque condition, la longueur moyenne de 200 neurites, le plus long de chaque cellule, est mesurée sur 3 cultures simultanées.

Les résultats sont représentés par 1, 2 ou 3 + selon que la stimulation est inférieure, égale ou supérieure à celle produite par la PREG.

| ***Stéroïde*** | ***Affinité*** | ***Activité*** | ***Pousse neuritique*** |
|---|---|---|---|
| | | | |
| *Prégnènolone (PREG)* | 100 | 100 | ++ |
| *3*β*-méthoxy-prégna-5-ène-20-one* | 100 | 100 | +++ |
| *3*β*-méthoxy-prégna-5-ène-20-one-17*α*-dichlorométhyle* | 53 | 113 | +++ |
| *3*β*-méthoxy-5*α*-prégnane-20-one* | 87 | 10 | +++ |
| *3*β*-méthoxy-5*α*-prégnane-20*β*-ol* | 65 | 65 | ++ |
| *PREG-16*α*-méthyle* | 80 | 70 | ++ |
| *PREG-16*β*-méthyle* | 63 | 67 | (++) |
| *3*β*-hydroxy-prégna-5,14-diène-20-one* | 102 | 50 | + |
| *PREG-16*α*,17*α*-époxy* | 41 | 54 | + |
| *PREG-16*α*,17*α*-méthylène* | 62 | 49 | + |
| *Prégna-5-ène-3*β*,20*β*-diol-20-acétate* | 60 | 108 | ++ |
| *3*β*-hydroxy-5*α*-prégnane-20-one-16*α*-méthyle* | 57 | 53 | (+) |

Ces résultats démontrent l'efficacité d'autres molécules dérivées de la prégnènolone pour stimuler la polymérisation des microtubules induite par MAP2, ou la pousse neuritique. Pour celles qui n'ont pas de dérivés 3β-méthyl, il est prévisible que cette dérivation maintiendra au moins leur activité.

## Revendications

1. Utilisation de la 3-méthoxy prégnénolone ou d'une molécule dérivée de la prégnénolone portant une fonction 3-méthoxy et incapable de se convertir en métabolites ou esters sulfate de prégnénolone pour la préparation d'un médicament pour traiter une maladie dégénérative du système nerveux, telle que la maladie choisie dans le groupe constitué de la maladie d'Alzheimer, la maladie de Parkinson, la perte de mémoire induite par l'âge, la perte de mémoire induite par la prise de substances, une lésion traumatique, une lésion cérébrale, une lésion de la moelle épinière, en particulier une compression médullaire, l'ischémie, la douleur, notamment la douleur névritique, la dégénérescence nerveuse et la sclérose en plaques, ladite molécule présentant la formule I : 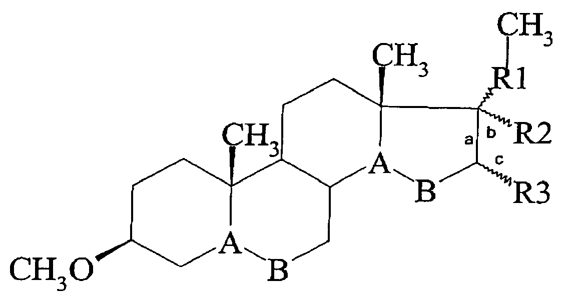 dans laquelle : 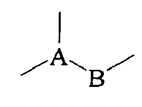 représente 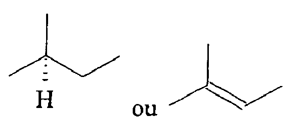
R1 = -CO- ; -CH(OH)- ou -CH(O-COCH₃)-
R2 = H ou CHCl₂,
R3 = H ou CH₃, ou
R2 et R3 forment ensemble un cycle :

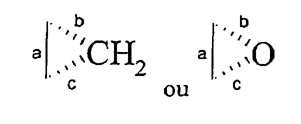

2. Utilisation selon la revendication 1, **caractérisée en ce que** ledit médicament comprend également un excipient permettant de formuler ladite molécule dérivée de prégnènolone pour traverser la barrière hémato-encéphalique.

3. Utilisation selon l'une des revendications 1 à 2, **caractérisée en ce que** ledit médicament se présente sous forme injectable.

4. Utilisation selon l'une des revendications 1 à 2, **caractérisée en ce que** ledit médicament se présente sous forme permettant une prise orale.

5. Utilisation selon l'une des revendications 1 à 4, **caractérisée en ce que** ladite molécule est la 3-méthoxy-PREG.

6. Utilisation selon l'une des revendications 1 à 4, **caractérisée en ce que** ladite molécule est la 3β-méthoxy-prégna-5-ène-20-one-17α-dichlorométhyle.

7. Utilisation selon l'une des revendications 1 à 4, **caractérisée en ce que** ladite molécule est la 3β-méthoxy-5α-prégnane-20-one.

8. Utilisation selon l'une des revendications 1 à 7, **caractérisé en ce que** ledit médicament comprend une quantité de 3-méthoxy-prégnénolone ou de molécule dérivée comprise entre 50 et 2500 mg.

9. 3β-méthoxy-prégna-5-ène-20-one en tant que médicament.

10. Composition pharmaceutique, comprenant de la 3-méthoxy-prégnénolone ou une molécule dérivée de la prégnénolone portant une fonction 3-méthoxy de formule générale I en tant que principe actif, et un excipient pharmaceutiquement acceptable.

11. Méthode *in vitro* pour augmenter la stabilisation et/ou induire la polymérisation des microtubules dans une cellule comprenant l'étape d'exposer ladite cellule à la présence de 3-méthoxy-prégnénolone, à une concentration d'environ 0,5 à 50 µM.

12. Méthode *in vitro* pour augmenter la pousse des neurites dans une cellule comprenant l'étape d'exposer ladite cellule à la présence de 3-méthoxy-prégnénolone, à une concentration d'environ 0,5 à 50 µM.

## Claims

1. Use of 3-methoxy-PREG or a molecule derived from pregnenolone which contains a 3-methoxy function and is incapable of being converted into pregnenolone metabolites or sulphate esters, for the preparation of a drug for treating a degenerative disease of the nervous system, such as the disease selected from the group consisting of Alzheimer's disease, Parkinson's disease, age-induced memory loss, memory loss induced by the taking of substances, a traumatic lesion, a cerebral lesion, a lesion of the spinal cord, in particular medullary compression, ischaemia, pain, notably neuritic pain, nerve degeneration, and multiple sclerosis, said molecule presenting formula I: 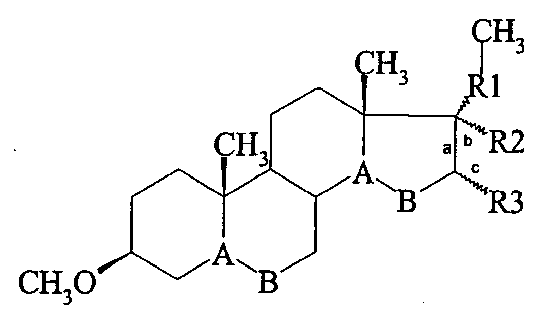 in which: 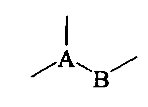 represents 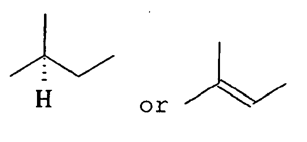
R1 = -CO- ; -CH(OH)- or -CH(O-COCH₃)-
R2 = H or CHCl₂,
R3 = H or CH₃, or
R2 and R3 together form a ring:

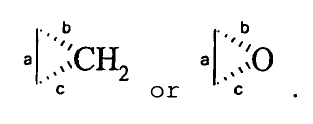

2. Use according to Claim 1, **characterised in that** said drug also comprises an excipient for formulating said molecule derived from pregnenolone to cross the blood-brain barrier.

3. Use according to any of Claims 1 to 2, **characterised in that** said drug is in an injectable form.

4. Use according to any of Claims 1 to 2, **characterised in that** said drug is in an oral form.

5. Use according to any of Claims 1 to 4, **characterised in that** said molecule is 3-methoxy-PREG.

6. Use according to any of Claims 1 to 4, **characterised in that** said molecule is 3β-methoxy-pregna-5-ene-20-one-17a-dichloromethyl.

7. Use according to any of Claims 1 to 4, **characterised in that** said molecule is 3β-methoxy-5α-pregnane-20-one.

8. Use according to any of Claims 1 to 7, **characterised in that** said drug comprises a quantity of 3-methoxy-pregnenolone or a derived molecule ranging between 50 and 2500 mg.

9. 3β-methoxy-pregna-5-ene-20-one as a drug.

10. A pharmaceutical composition, comprising 3-methoxy-pregnenolone or a molecule derived from pregnenolone which contains a 3-methoxy function of general formula I as an active ingredient, and a pharmaceutically acceptable excipient.

11. An *in vitro* method for increasing stabilisation and/or inducing polymerisation of microtubules in a cell, comprising the step of exposing said cell to the presence of 3-methoxy-pregnenolone at a concentration of approximately 0.5 to 50 µM.

12. An *in vitro* method for increasing neuritic sprouting in a cell, comprising the step of exposing said cell to the presence of 3-methoxy-pregnenolone at a concentration of approximately 0.5 to 50 µM.

## Patentansprüche

1. Verwendung von 3-Methoxy-Pregnenolon der eines Moleküls, das eine Pregnenolon-Ableitung ist, eine 3-Methoxy-Funktion trägt und nicht in der Lage ist, sich in Pregnenolon-Metabolite oder - sulfatester umzuwandeln, für die Zubereitung eines Arzneimittels zur Behandlung einer degenerativen Erkrankung des Nervensystems, wie die Krankheit, die aus der Gruppe ausgewählt ist, die von der Alzheimer-Krankheit, der Parkinson-Krankheit, dem altersbedingten Gedächtnisverlust, dem durch die Einnahme von Substanzen bedingten Gedächtnisverlust, einer traumatischen Verletzung, einer Verletzung des Gehirns, einer Verletzung des Rückenmarks, insbesondere einer Rückenmarkkompression, der Ischämie, dem Schmerz, vor allem dem Neuralschmerz, der Nervendegeneration und der Multiplen Sklerose gebildet wird, wobei das besagte Molekül die Formel I aufweist: 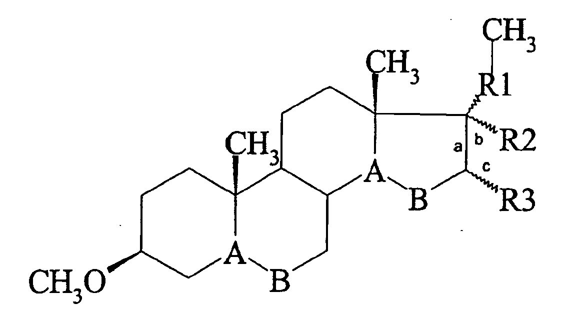 wobei: 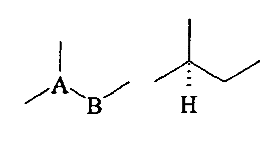 darstellt oder 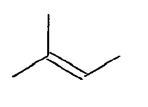
R1 = -CO-; -CH(OH)- oder -CH(O-COCH₃)-
R2 = H oder CHCl₂,
R3 = H oder CH₃, oder
R2 und R3 gemeinsam einen Ring bilden

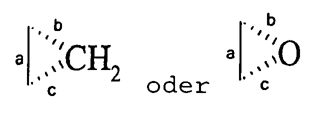

2. Verwendung nach Anspruch 1, **dadurch gekennzeichnet, dass** das besagte Arzneimittel ebenfalls einen Exzipienten umfasst, der es erlaubt, das besagte, vom Pregnenolon abgeleitete Molekül zu formulieren, um die Blut-Hirn-Schranke zu durchqueren.

3. Verwendung nach einem der Ansprüche 1 bis 2, **dadurch gekennzeichnet, dass** sich das besagte Arzneimittel in injizierbarer Form darstellt.

4. Verwendung nach einem der Ansprüche 1 bis 2, **dadurch gekennzeichnet, dass** sich das besagte Arzneimittel in einer Form darstellt, die eine orale Einnahme erlaubt.

5. Verwendung nach einem der Ansprüche 1 bis 4, **dadurch gekennzeichnet, dass** das besagte Molekül 3-Methoxy-PREG ist.

6. Verwendung nach einem der Ansprüche 1 bis 4, **dadurch gekennzeichnet, dass** das besagte Molekül 3β-Methoxy-pregna-5-ene-20-one-17α-dichlormethyl ist.

7. Verwendung nach einem der Ansprüche 1 bis 4, **dadurch gekennzeichnet, dass** das besagte Molekül 3β-Methoxy-5α-pregnan-20-on ist.

8. Verwendung nach einem der Ansprüche 1 bis 7, **dadurch gekennzeichnet, dass** das besagte Arzneimittel eine Menge von 3-Methoxy-pregnenolon oder eines abgeleiteten Moleküls einschließlich zwischen 50 und 2500 mg umfasst.

9. 3β-Methoxy-pregna-5-ene-20-on als Arzneimittel.

10. Pharmazeutische Zusammensetzung, 3-Methoxy-pregnenolon oder ein vom Pregnenolon abgeleitetes Molekül umfassend, das eine 3-Methoxy-Funktion der allgemeinen Formel I als Wirkstoffprinzip trägt, und einen pharmazeutisch akzeptablen Exzipienten.

11. *In vitro*-Methode zur Erhöhung der Stabilisierung und/oder Induzierung der Polymerisation von Mikrotubuli in einer Zelle, den Schritt umfassend, die besagte Zelle der Präsenz von 3-Methoxy-pregnenolon in einer Konzentration von zirka 0,5 bis 50 µM auszusetzen.

12. *In vitro*-Methode zur Erhöhung des Neuritenwachstums in einer Zelle, den Schritt umfassend, die besagte Zelle der Präsenz von 3-Methoxy-pregnenolon in einer Konzentration von zirka 0,5 bis 50 µM auszusetzen.
